# EUROPEAN PATENT SPECIFICATION

(11) **EP 4 194 037 B1**
(45) Date of publication and mention of the grant of the patent: **30.10.2024**
(21) Application number: 22211675.8
(22) Date of filing: 06.12.2022
(51) Int. Cl.: A61M 11/04

(54) **ATOMIZER AND ELECTRONIC ATOMIZING DEVICE**
ZERSTÄUBER UND ELEKTRONISCHE ZERSTÄUBUNGSVORRICHTUNG
ATOMISEUR ET DISPOSITIF D'ATOMISATION ÉLECTRONIQUE

(30) Priority: 08.12.2021 CN 202111495547
(43) Date of publication of application: 14.06.2023
(73) Proprietor: Shenzhen Smoore Technology Limited, Shenzhen, Guangdong 518102 (CN)
(72) Inventor: LI, Xiaoping, Shenzhen, 518102 (CN)
(74) Representative: Manitz Finsterwald Patent- und Rechtsanwaltspartnerschaft mbB

(56) References cited:
- WO-A1-2020/259692
- CN-A- 112 586 806
- US-A1- 2019 191 772
- US-B2- 9 883 698

## Description

### TECHNICAL FIELD

The present invention relates to the field of atomizing technologies, and in particular, to an atomizer and an electronic atomizing device.

### BACKGROUND

Aerosol is a colloidal dispersion system formed by small solid or liquid particles dispersed and suspended in a gas medium. Since the aerosol is absorbable by the human body through the respiratory system, a novel alternative absorption manner is provided for the user. For example, atomizing devices that can generate aerosols by baking and heating herbal or paste-like aerosol-forming substrates are applied to different fields, to deliver inhalable aerosols for the users, leading to replacement of conventional product forms and absorption manners.

Generally, an electronic atomizing device heats and vaporizes the aerosol-forming substrate through an atomizer. However, an air inlet of the atomizer is provided on a bottom surface, condensate may be formed when the aerosol inside the atomizer flows back, and the condensate is likely to overflow from the air inlet under the action of its own gravity, resulting in corrosion damage to electronic components such as a cell at the bottom of the atomizer. In addition, the internal airway of a conventional atomizer is relatively long, and the inner wall of the airway provides a relatively long bearing surface for aerosol that flows back, which is likely to result in formation of more condensate. Therefore, the conventional atomizer is prone to formation of a large amount of condensate that overflows. Related technologies are known from CN 112 586 806 A, US 2019/191772 A1, and US 9 883 698 B2.

### SUMMARY

In view of this, it is necessary to provide an atomizer and an electronic atomizing device to resolve the problem that the conventional atomizer is prone to formation of a large amount of condensate that overflows. The present invention is set out in the appended claims.

An atomizer is provided, including: a housing, wherein an air outlet channel is formed in the housing; and an atomizing assembly assembled in the housing and including a base and an atomizing core inserted in the base, wherein the base includes a top wall facing the air outlet channel and a side wall intersecting with and connected to the top wall; wherein an atomizing channel in communication with the air outlet channel is defined between the side wall and the atomizing core, the atomizing channel is located in an axial direction of the air outlet channel, and an air inlet in communication with the atomizing channel is provided on the side wall.

In the foregoing atomizer, the atomizing channel is defined between the side wall of the base and the atomizing core, and the atomizing channel is located in the axial direction of the air outlet channel, so that external air directly enters the atomizing channel through the air inlet on the side wall and then can enter the air outlet channel in the axial direction of the air outlet channel, which is equivalent to that an airflow pathway inside the atomizer is L-shaped, and the pathway is relatively simple and short. Therefore, the problem that the airflow pathway is excessively long and absorbs a large amount of aerosol and generates a large amount of condensate is avoided, thereby reducing the generated condensate. In addition, the air inlet of the atomizer is located on the side wall of the base rather than a bottom wall of the base, so that condensate generated in an internal airway may not directly flow out through the air inlet on the side wall, thereby preventing the leakage of the condensate.

In this way, the atomizer provided by this application not only can prevent condensate from leaking through the air inlet, but also can prevent generation of excessive condensate, thereby reducing the risk of the overflow of the condensate and reducing the amount of condensate leaking from the atomizer. In addition, the airflow pathway inside the atomizer provided by this application is relatively short, so that an external airflow can quickly enter the atomizing channel to carry the atomized aerosol, thereby improving the atomizing amount of the aerosol-forming substrate.

In an embodiment, the atomizing core includes an atomizing surface parallel to the axial direction of the air outlet channel, the atomizing surface is relatively spaced apart from the side wall, and the atomizing channel is defined between the atomizing surface and the side wall.

In an embodiment, the air inlet directly faces the atomizing surface.

In an embodiment, the atomizing assembly further includes an electrode, one end of the electrode is electrically connected to a heating member on the atomizing core, and the other end of the electrode penetrates through the side wall and is relatively exposed on an outer surface of the side wall.

In an embodiment, the electrode penetrates through the side wall in a direction intersecting with the axial direction of the air outlet channel.

In an embodiment, a docking cavity is defined between the housing and the outer surface of the side wall, where the docking cavity is configured to accommodate a docking convex portion of a cell base.

In an embodiment, the base includes a base body and a holder, the atomizing core and the holder are both inserted in the base body, one end of the holder abuts against the atomizing core, the other end of the holder is the side wall and is provided with the air inlet, and the electrode fixedly penetrates through the end of the holder provided with the air inlet.

In an embodiment, the atomizing core includes a liquid absorbing surface away from the atomizing surface, a liquid storage cavity is formed inside the housing, a liquid inlet channel in communication with the liquid storage cavity is provided on the base body, and the liquid absorbing surface is in fluid communication with the liquid inlet channel.

In an embodiment, the liquid storage cavity surrounds a periphery of the air outlet channel, and the liquid inlet channel is located on a surface of the atomizing core away from the air inlet and extends in a direction parallel to the axial direction of the air outlet channel.

In an embodiment, the atomizer further includes a magnetic member, the base includes a bottom wall opposite to the top wall, and the magnetic member is assembled on the bottom wall.

An electronic atomizing device is provided including the foregoing atomizer.

### BRIEF DESCRIPTION OF THE DRAWINGS

FIG. 1 is a cross-sectional view of an atomizer according to an embodiment of the present invention;
FIG. 2 is a perspective view of an atomizing assembly of the atomizer shown in FIG. 1;
FIG. 3 is a perspective exploded view of the atomizing assembly shown in FIG. 2; and
FIG. 4 is a cross-sectional view of an electronic atomizing device according to an embodiment of the present invention.

Reference Numerals: 100 - Atomizer; 10 - Housing; 11 - Air outlet channel; 12 - Outer housing; 13 - Liquid storage cavity; 14 - Inner housing; 30 - Atomizing assembly; 32 - Base; 321 - Top wall; 323 - Side wall; 324 - Bottom wall; 325 - Base body; 326 - Air passing opening; 327 - Holder; 33 - Air inlet; 34 - Atomizing core; 35 - Atomizing channel; 341 - Atomizing surface; 343 - Liquid absorbing surface; 36 - Electrode; 37 - Liquid inlet channel; 40 - Docking cavity; 50 - Magnetic member; 200 - Electronic atomizing device; 210 - Cell base; and 211 - Docking convex portion.

### DETAILED DESCRIPTION

To make the foregoing objects, features, and advantages of the present invention more comprehensible, detailed description is made to specific implementations of the present invention below with reference to the accompanying drawings. In the following description, many specific details are described to give a full understanding of the present invention.

The present invention is defined by the appended claims.

In the description of the present invention, it should be understood that, orientation or position relationships indicated by terms such as "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", and "circumferential" are orientation or position relationship shown based on the accompanying drawings, and are merely used for describing the present invention and simplifying the description, rather than indicating or implying that the mentioned apparatus or element should have a particular orientation or be constructed and operated in a particular orientation, and therefore, should not be construed as a limitation to the present invention.

In addition, the terms "first" and "second" are used for descriptive purposes only and are not to be construed as indicating or implying relative importance or implicitly indicating the number of technical features indicated. Therefore, features defining "first" and "second" can explicitly or implicitly include at least one of the features. In the description of the present invention, unless otherwise explicitly defined, "a plurality of" means at least two, for example, two, three, and the like.

In the present invention, unless otherwise explicitly specified and defined, terms such as "mounted", "connected", "connection", and "fixed" should be understood in a broad sense. For example, the connection may be a fixed connection, a detachable connection, or an integral connection; or the connection may be a mechanical connection or an electrical connection; or the connection may be a direct connection, an indirect connection through an intermediate medium, or internal communication between two elements or mutual action relationship between two elements, unless otherwise explicitly specified. A person of ordinary skill in the art may understand the specific meanings of the foregoing terms in the present invention according to specific situations.

In the present invention, unless otherwise clearly specified and limited, that a first feature is "above" or "below" a second feature may be that the first and the second features are in contact with each other directly, or the first and the second features are in contact with each other indirectly by using an intermediate medium. In addition, that the first feature is "above", "over", or "on" the second feature may indicate that the first feature is directly above or obliquely above the second feature, or may merely indicate that the horizontal position of the first feature is higher than that of the second feature. That the first feature is "below", "under", and "beneath" the second feature may be that the first feature is right below the second feature or at an inclined bottom of the second feature, or may merely indicate that the horizontal position of the first feature is lower than that of the second feature.

It should be noted that, when an element is referred to as "being fixed to" or "being arranged on" another element, the element may be directly on the other element, or an intermediate element may be present. When an element is considered to be "connected to" another element, the element may be directly connected to the other element, or an intermediate element may also be present. The terms "vertical", "horizontal", "upper", "lower", "left", "right", and similar expressions used in this specification are only for purposes of illustration but are not intended to indicate a unique implementation.

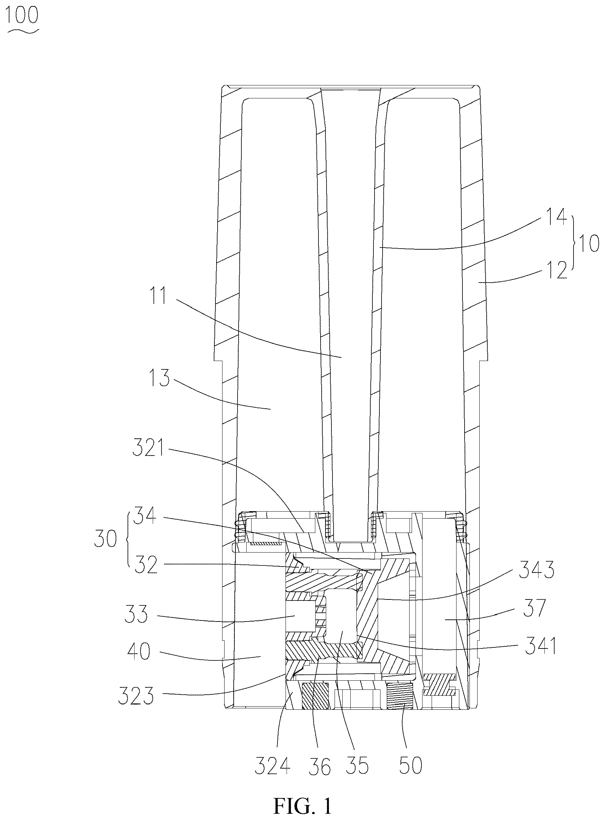

FIG. 1 is a cross-sectional view of an atomizer according to an embodiment of the present invention. Referring to FIG. 1, an embodiment of the present invention provides an atomizer 100, which is configured to heat and atomize an aerosol-forming substrate to form aerosol delivered to a user.

The atomizer 100 includes a housing 10 and an atomizing assembly 30. An air outlet channel 11 is formed in the housing 10. The atomizing assembly 30 is assembled in the housing 10 and includes a base 32 and an atomizing core 34 inserted in the base 32.

The base 32 includes a top wall 321 facing the air outlet channel 11 and a side wall 323 intersecting with and connected to the top wall 321. An atomizing channel 35 in communication with the air outlet channel 11 is defined between the side wall 323 of the base 32 and the atomizing core 34. The atomizing channel 35 is located in an axial direction of the air outlet channel 11, and an air inlet 33 in communication with the atomizing channel 35 is provided on the side wall 323. During operation of the atomizer 100, an aerosol-forming substrate is adsorbed in the atomizing core 34, and after the atomizing core 34 is heated, the aerosol-forming substrate is atomized into aerosol and enters the atomizing channel 35. Then an airflow entered from the air inlet 33 flows through the atomizing channel 35, and carries the aerosol to flow out of the air outlet channel 11, so as to deliver the atomized aerosol to the user.

In this embodiment, the atomizing channel 35 is defined between the side wall 323 of the base 32 and the atomizing core 34, and the atomizing channel 35 is located in the axial direction of the air outlet channel 11, so that external air directly enters the atomizing channel 35 through the air inlet 33 on the side wall 323 and then can enter the air outlet channel 11 in the axial direction of the air outlet channel 11, which is equivalent to that an airflow pathway inside the atomizer 100 is L-shaped, and the pathway is relatively simple and short. Therefore, the problem that the airflow pathway is excessively long and absorbs a large amount of aerosol and generates a large amount of condensate is avoided, thereby reducing the generated condensate. In addition, the air inlet 33 of the atomizer 100 is located on the side wall 323 of the base 32 rather than a bottom wall 324 of the base 32, so that condensate generated in an internal airway may not directly flow out through the air inlet 33 on the side wall, thereby preventing the leakage of the condensate.

In this way, the atomizer 100 provided by this embodiment not only can prevent condensate from leaking through the air inlet 33, but also can prevent generation of excessive condensate, thereby reducing the risk of the leakage of the condensate, and reducing the amount of condensate leaking from the atomizer 100. In addition, the airflow pathway inside the atomizer 100 provided in this application is relatively short, so that an external airflow can quickly enter the atomizing channel 35 to carry the atomized aerosol, thereby improving the atomizing amount of the aerosol-forming substrate.

Further, the atomizing core 34 includes an atomizing surface 341 parallel to the axial direction of the air outlet channel 11, that is, the atomizing core 34 is laterally arranged. The atomizing surface 341 is relatively spaced apart from the side wall 323, and the atomizing channel 35 is defined between the atomizing surface 341 and the side wall 323, so that the formed atomizing channel 35 has a linear shape with a simple structure and a short pathway, thereby reducing the formation of condensate while improving the atomizing amount of the aerosol-forming substrate.

Specifically, the air inlet 33 provided on the side wall 323 directly faces the atomizing surface 341, so that the air inlet 33 is in direct communication with the atomizing channel 35 between the side wall 323 and the atomizing surface 341, and air flowing in an axial direction of the air inlet 33 can directly enter the atomizing channel 35 without making a turn before entering the atomizing channel 35, thereby shortening the airflow pathway.

In some embodiments, the atomizing assembly 30 further includes an electrode 36. The atomizing core 34 further includes a heating member. One end of the electrode 36 is electrically connected to the heating member, and the other end of the electrode 36 penetrates through the side wall 323 and is relatively exposed on an outer surface of the side wall 323. It is equivalent to that, not only the air inlet 33 is provided on the side wall 323 of the base 32, but also the electrode 36 penetrates through the side wall 323, so that the electrode 36 is fixedly mounted through the side wall 323. In addition, the electrode 36 is relatively exposed on the outer surface of the side wall 323, so that the electrode 36 can be used as a docking end of the heating member of the atomizing core 34 and that is electrically connected to a cell base 210. Therefore, the docking end has a specific area, even if there is a slight relative displacement between the atomizer 100 and the cell base 210 in a mounting direction, the electrical contact between the electrode 36 on the side wall 323 and the cell base 210 can be ensured, thereby improving the stability of power supply.

Further, the electrode 36 penetrates through the side wall 323 in a direction intersecting with the axial direction of the air outlet channel 11, that is, the electrode 36 is laterally arranged. In this way, after the atomizer 100 and the cell base 210 are vertically mounted in a docked manner, the laterally arranged electrode 36 may still be in electrical contact with the cell base 210 on the outer surface of the side wall 323, even if the cell base 210 is slightly misaligned with the atomizer 100 in the mounting direction.

In some embodiments, a docking cavity 40 is defined between the housing 10 and the outer surface of the side wall 323, and the docking cavity 40 is configured to accommodate a docking convex portion 211 on the cell base 210. During assembly of the atomizer 100 and the cell base 210, the docking convex portion 211 on the cell base 210 is inserted into the docking cavity 40, so that the electrode 36 relatively exposed on the outer surface of the side wall 323 can be in electrical contact with a docking terminal on the docking convex portion 211 through the docking cavity 40, thereby achieving the electrical connection between the heating member on the atomizing core 34 and the cell base 210. In addition, the docking cavity 40 accommodates the docking convex portion 211 to limit the docking convex portion 211, so that the docking convex portion 211 is prevented from being separated from the side wall 323, thereby ensuring that the electrode 36 is electrically connected to the cell base 210 effectively after the docking convex portion 211 comes in contact with the side wall 323, and further improving the reliability of power supply.

Specifically, the docking cavity 40 can limit the docking convex portion 211 in a first direction perpendicular to the air outlet channel 11 and a second direction perpendicular to the first direction, so as to prevent the docking convex portion 211 from being displaced in the thickness and width directions of the atomizer 100 and improve the reliability of contact between the docking convex portion 211 and the side wall 323, thereby improving the user experience.

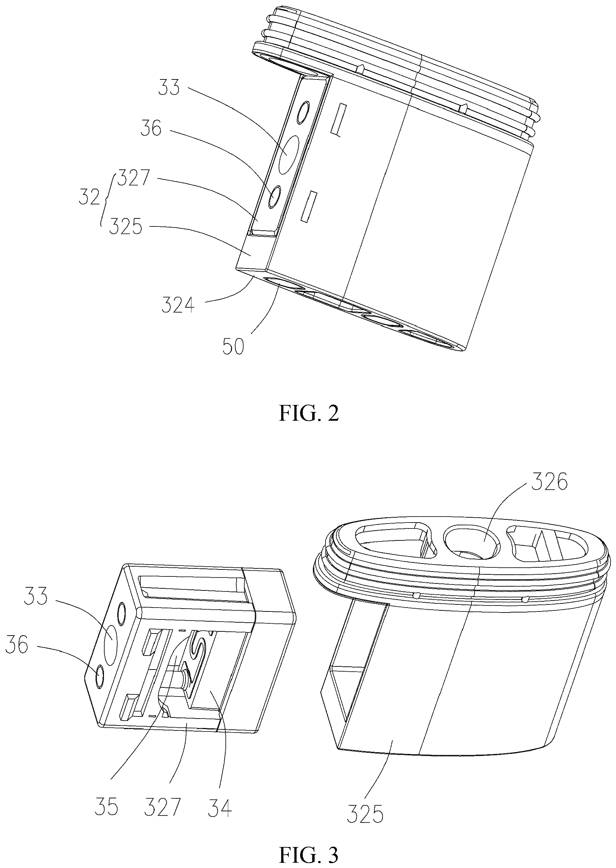

FIG. 2 is a perspective of an atomizing assembly of the atomizer shown in FIG. 1. FIG. 3 is a perspective exploded view of the atomizing assembly shown in FIG. 2.

Referring to FIG. 2 and FIG. 3, in some embodiments, the base 32 includes a base body 325 and a holder 327. The atomizing core 34 and the holder 327 are both inserted in the base body 325. One end of the holder 327 abuts against the atomizing core 34, the other end of the holder 327 is the side wall 323 and is provided with the air inlet 33, and the electrode 36 fixedly penetrates through the end of the holder 327 provided with the air inlet 33. For the ease of assembling the atomizing core 34 and forming the atomizing channel 35, the base 32 is divided into the base body 325 and the holder 327, the atomizing core 34 is inserted in the base body 325, and the holder 327 is further sleeved on the base body 325 and abuts against the atomizing core 34, so as to fix the atomizing core 34. In addition, an end of the holder 327 away from the atomizing core 34 is constructed as the side wall 323 and is provided with the air inlet 33, so that the atomizing channel 35 is formed between the holder 327 and the atomizing core 34. Moreover, the holder 327 is configured to fix a cell on the base 32.

Further, an air passing opening 326 in communication with the atomizing channel 35 and the air outlet channel 11 is provided on the base body 325, so as to allow airflow to flow from the atomizing channel 35 to the air outlet channel 11. In addition, a through groove in communication with the atomizing channel 35 and the air passing opening 326 is formed on the holder 327, so that the electrode 36 can be assembled through the holder 327 without affecting the communication between the atomizing channel 35 between the holder 327 and the atomizing core 34 and the air outlet channel 11.

Referring to FIG. 1, specifically, the atomizing core 34 includes a liquid absorbing surface 343 away from the atomizing surface 341. A liquid storage cavity 13 is formed inside the housing 10, a liquid inlet channel 37 in communication with the liquid storage cavity 13 is provided on the base body 325, and the liquid absorbing surface 343 is in fluid communication with the liquid inlet channel 37. It is equivalent to that, the liquid inlet channel 37 is provided on the base body 325, and the aerosol-forming substrate in the liquid storage cavity 13 is guided to the liquid absorbing surface 343 on the atomizing core 34, so that the aerosol-forming substrate is adsorbed and stored inside the atomizing core 34.

Optionally, the liquid storage cavity 13 surrounds a periphery of the air outlet channel 11, the liquid inlet channel 37 is located on a surface of the atomizing core 34 away from the air inlet 33, and the liquid inlet channel 37 extends in a direction parallel to the axial direction of the air outlet channel 11. It is equivalent to that, one side of the base 32 is provided with the air inlet 33, and the other side of the base 32 is provided with the liquid inlet channel 37, the aerosol-forming substrate in the annular liquid storage cavity 13 is guided to the atomizing core 34 through the liquid inlet channel 37 on one side away from the air inlet 33. In addition, when the atomizing core 34 is normally held by the user, the liquid inlet channel 37 extends in a vertical direction, and the aerosol-forming substrate in the liquid storage cavity 13 is allowed to be in contact with the liquid absorbing surface 343 of the atomizing core 34 after flowing toward the liquid inlet channel 37.

In some embodiments, the housing 10 includes an outer housing 12 and an inner housing 14 inserted in the outer housing 12. The inner housing 14 has the air outlet channel 11, and the liquid storage cavity 13 is defined between the inner housing 14 and the outer housing 12. The outer housing 12 is further provided with an accommodation cavity located on the same side of the liquid storage cavity 13 and the air outlet channel 11, and the atomizing assembly 30 is provided in the accommodation cavity and seals the liquid storage cavity 13. The atomizing assembly 30 is inserted in the outer housing 12, an air outlet of the atomizing assembly 30 is in communication with the air outlet channel 11 formed on the inner housing 14. In addition, the atomizing assembly 30 blocks an opening of the liquid storage cavity 13, so that the assembly of the atomizing assembly 30 and the housing 10 is completed.

In some embodiments, the atomizer 100 further includes a magnetic member 50. The base 32 includes a bottom wall 324 arranged opposite to the top wall 321, and the magnetic member 50 is assembled on the bottom wall 324, so that the atomizer 100 can be attracted and fixed to the cell base 210 through the magnetic member 50, thereby improving the mounting reliability between the atomizer 100 and the cell base 210.

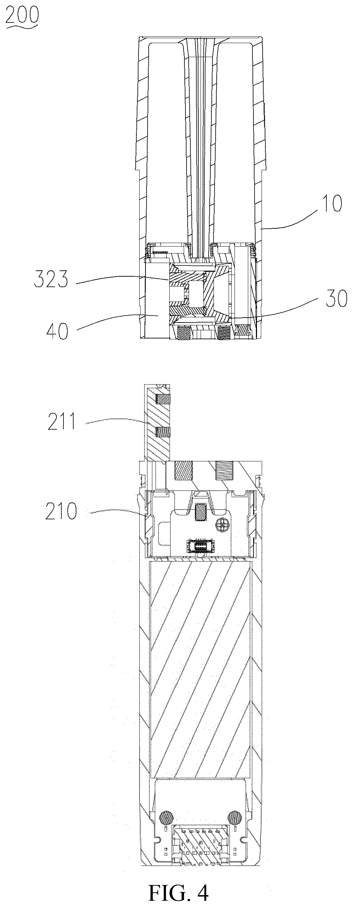

FIG. 4 is a cross-sectional view of an electronic atomizing device 200 according to an embodiment of the present invention. Referring to FIG. 4, the electronic atomizing device 200 includes the atomizer 100. The atomizer 100 includes a housing 10 and an atomizing assembly 30. An air outlet channel 11 is formed in the housing 10. The atomizing assembly 30 is assembled in the housing 10 and includes a base 32 and an atomizing core 34 inserted in the base 32. The base 32 includes a top wall 321 facing the air outlet channel 11 and a side wall 323 intersecting with and connected to the top wall 321. An atomizing channel 35 in communication with the air outlet channel 11 is defined between the side wall 323 of the base 32 and the atomizing core 34, the atomizing channel 35 is located in an axial direction of the air outlet channel 11, and an air inlet 33 in communication with the atomizing channel 35 is provided on the side wall 323. In this way, the atomizing channel 35 is defined between the side wall 323 of the base 32 and the atomizing core 34, and the atomizing channel 35 is located in the axial direction of the air outlet channel 11, so that external air directly enters the atomizing channel 35 through the air inlet 33 on the side wall 323 and then can enter the air outlet channel 11 in the axial direction of the air outlet channel 11, which is equivalent to that an airflow pathway inside the atomizer 100 is L-shaped, and the pathway is relatively simple and short, so that the airflow pathway is prevented from generating a large amount of condensate after absorbing a large amount of aerosol because of being excessively long, thereby reducing the formation of condensate.

In addition, the air inlet 33 of the atomizer 100 is located on the side wall 323 of the base 32 rather than a bottom wall 324 of the base 32, so that condensate generated in an internal airway may not directly flow out through the air inlet 33 on the side wall, thereby preventing the leakage of the condensate. Therefore, the atomizer 100 provided in this embodiment not only can prevent condensate from leaking through the air inlet 33, but also can prevent generation of excessive condensate, thereby reducing the risk of condensate leakage, and reducing the amount of condensate leaking from the atomizer 100. In addition, the airflow pathway inside the atomizer 100 provided in this embodiment is relatively short, so that an external airflow can quickly enter into the atomizing channel 35 to carry the atomized aerosol, thereby improving the atomizing amount of the aerosol-forming substrate.

In some embodiments, the electronic atomizing device 200 further includes a cell base 210. The cell base 210 includes a main body and a docking convex portion 211 protruding from the main body. The docking convex portion 211 includes a docking terminal, and the docking terminal can be in electrical contact with the electrode 36 when the docking convex portion 211 is docked with the side wall 323, so as to achieve the electrical connection between the atomizer 100 and the cell base 210.

Further, the docking cavity 40 is formed in the atomizer 100, and the docking convex portion 211 is inserted in the docking cavity 40. In this way, the docking cavity 40 accommodates the docking convex portion 211 to limit and fix the docking convex portion 211, so that the docking convex portion 211 is prevented from being separated from the side wall 323, thereby ensuring that the electrode 36 is electrically connected to the cell base 210 effectively after the docking convex portion 211 is in contact with the side wall 323, and further improving the reliability of power supply.

Specifically, the docking cavity 40 limits the docking convex portion 211 in a first direction perpendicular to the air outlet channel 11 and a second direction perpendicular to the first direction, so as to prevent the docking convex portion 211 from being displaced in the thickness and width directions of the atomizer 100 and improve the reliability of contact between the docking convex portion 211 and the side wall 323, thereby improving the user experience.

In some embodiments, an air inlet channel communicating between the outside and the air inlet 33 is formed inside the docking convex portion 211, the external airflow passes through the air inlet channel in the docking convex portion 211 and then enters the air inlet 33 on the side wall 323, and finally flows inside the atomizer 100. In this way, air is supplied to the air inlet 33 through the docking convex portion 211, thus achieving air intake from a side of the atomizer 100. In addition, since the air inlet channel in the docking convex portion 211 is located on a side of the air inlet 33 rather than being located at the bottom of the air inlet 33, when a part of the aerosol in the atomizer 100 flows back to form condensate, the condensate cannot easily enter the docking convex portion 211 on the side through the air inlet 33, which can effectively prevent electronic components inside the cell base 210 from corrosion damage caused by the condensate, thereby prolonging the service life of the electronic atomizing device 200.

The technical features in the foregoing embodiments may be randomly combined. For concise description, not all possible combinations of the technical features in the embodiments are described. However, provided that combinations of the technical features do not conflict with each other, the combinations of the technical features are considered as falling within the scope described in this specification.

The foregoing embodiments only describe several implementations of the present disclosure, and the description is specific and detailed, but cannot therefore be understood as a limitation to the patent scope of the present invention. It should be noted that, a person of ordinary skill in the art may further make some variations and improvements without departing from the concept of the present invention as set out in the claims, and the variations and improvements belong to the protection scope of the present invention. Therefore, the protection scope of the patent of the present invention shall be subject to the appended claims.

## Claims

1. An atomizer (100), comprising:
a housing (10), wherein an air outlet channel (11) is formed in the housing (10); and
an atomizing assembly (30) assembled in the housing (10) and comprising a base (32) and an atomizing core (34) inserted in the base (32), wherein the base (32) comprises a top wall (321) facing the air outlet channel (11) and a side wall (323) intersecting with and connected to the top wall (321);
wherein an atomizing channel (35) in communication with the air outlet channel (11) is defined between the side wall (323) and the atomizing core (34), the atomizing channel (35) is located in an axial direction of the air outlet channel (11), and an air inlet (33) in communication with the atomizing channel (35) is provided;
wherein the atomizing core (34) comprises an atomizing surface (341) parallel to the axial direction of the air outlet channel (11), the atomizing surface (341) is relatively spaced apart from the side wall (323), and the atomizing channel (35) is defined between the atomizing surface (341) and the side wall (323);
wherein the atomizing assembly (30) further comprises an electrode (36), the atomizing core (34) further comprises a heating member, one end of the electrode (36) is electrically connected to the heating member, and the other end of the electrode (36) penetrates through the side wall (323) and is relatively exposed on an outer surface of the side wall (323);
wherein
the base (32) comprises a base body (325) and a holder (327), the atomizing core (34) and the holder (327) are both inserted in the base body (325), one end of the holder (327) abuts against the atomizing core (34), the other end of the holder (327) is the side wall (323)
**characterized in that**
the air inlet (33) in communication with the atomizing channel (35) is provided on the side wall (323) and the electrode (36) fixedly penetrates through the end of the holder (327) provided with the air inlet (33).

2. The atomizer (100) according to claim 1, wherein the air inlet (33) directly faces the atomizing surface (341).

3. The atomizer (100) according to claim 1, wherein the electrode (36) penetrates through the side wall (323) in a direction intersecting with the axial direction of the air outlet channel (11).

4. The atomizer (100) according to claim 1, wherein a docking cavity (40) is defined between the housing (10) and the outer surface of the side wall (323), wherein the docking cavity (40) is configured to accommodate a docking convex portion (211) of a cell base (210).

5. The atomizer (100) according to claim 1, wherein the atomizing core (34) comprises a liquid absorbing surface (343) away from the atomizing surface (341), a liquid storage cavity (13) is formed inside the housing (10), a liquid inlet channel (37) in communication with the liquid storage cavity (13) is provided on the base body (325), and the liquid absorbing surface (343) is in fluid communication with the liquid inlet channel (37).

6. The atomizer (100) according to claim 5, wherein the liquid storage cavity (13) surrounds a periphery of the air outlet channel (11), and the liquid inlet channel (37) is located on a surface of the atomizing core (34) away from the air inlet (33) and extends in a direction parallel to the axial direction of the air outlet channel (11).

7. The atomizer (100) according to any one of claims 1 to 6, further comprising a magnetic member (50), wherein the base (32) comprises a bottom wall (324) opposite to the top wall (321), and the magnetic member (50) is assembled on the bottom wall (324).

8. An electronic atomizing device (200), comprising the atomizer (100) according to any one of claims 1 to 7.

## Patentansprüche

1. Zerstäuber (100), umfassend:
ein Gehäuse (10), wobei ein Luftauslasskanal (11) in dem Gehäuse (10) ausgebildet ist; und
eine Zerstäubungsanordnung (30), die in dem Gehäuse (10) montiert ist und eine Basis (32) und einen Zerstäubungskern (34) umfasst, der in die Basis (32) eingesetzt ist, wobei die Basis (32) eine obere Wand (321), die dem Luftauslasskanal (11) zugewandt ist, und eine Seitenwand (323), die sich mit der oberen Wand (321) schneidet und mit dieser verbunden ist,
umfasst;
wobei ein Zerstäubungskanal (35), der mit dem Luftauslasskanal (11) in Verbindung steht, zwischen der Seitenwand (323) und dem Zerstäubungskern (34) definiert ist, der Zerstäubungskanal (35) in einer axialen Richtung des Luftauslasskanals (11) angeordnet ist und ein Lufteinlass (33), der mit dem Zerstäubungskanal (35) in Verbindung steht, vorgesehen ist;
wobei der Zerstäubungskern (34) eine Zerstäubungsfläche (341) parallel zu der axialen Richtung des Luftauslasskanals (11) umfasst, die Zerstäubungsfläche (341) relativ zu der Seitenwand (323) beabstandet ist und der Zerstäubungskanal (35) zwischen der Zerstäubungsfläche (341) und der Seitenwand (323) definiert ist;
wobei die Zerstäubungsanordnung (30) ferner eine Elektrode (36) umfasst, der Zerstäubungskern (34) ferner ein Heizelement umfasst, ein Ende der Elektrode (36) elektrisch mit dem Heizelement verbunden ist und das andere Ende der Elektrode (36) durch die Seitenwand (323) dringt und an einer Außenfläche der Seitenwand (323) relativ freiliegt;
wobei
die Basis (32) einen Basiskörper (325) und einen Halter (327) umfasst, der Zerstäubungskern (34) und der Halter (327) beide in den Basiskörper (325) eingesetzt sind, ein Ende des Halters (327) an dem Zerstäubungskern (34) anliegt und das andere Ende des Halters (327) die Seitenwand (323) ist, **dadurch gekennzeichnet, dass**
der Lufteinlass (33), der mit dem Zerstäubungskanal (35) in Verbindung steht, an der Seitenwand (323) vorgesehen ist und die Elektrode (36) fest durch das Ende des Halters (327), das mit dem Lufteinlass (33) versehen ist, dringt.

2. Zerstäuber (100) nach Anspruch 1, wobei der Lufteinlass (33) direkt der Zerstäubungsfläche (341) zugewandt ist.

3. Zerstäuber (100) nach Anspruch 1, wobei die Elektrode (36) durch die Seitenwand (323) in einer Richtung dringt, die sich mit der axialen Richtung des Luftauslasskanals (11) schneidet.

4. Zerstäuber (100) nach Anspruch 1, wobei ein Andockhohlraum (40) zwischen dem Gehäuse (10) und der Außenfläche der Seitenwand (323) definiert ist, wobei der Andockhohlraum (40) konfiguriert ist, um einen konvexen Andockabschnitt (211) einer Zellenbasis (210) aufzunehmen.

5. Zerstäuber (100) nach Anspruch 1, wobei der Zerstäubungskern (34) eine flüssigkeitsabsorbierende Fläche (343) weg von der Zerstäubungsfläche (341) umfasst, ein Flüssigkeitsspeicherhohlraum (13) innerhalb des Gehäuses (10) ausgebildet ist, ein Flüssigkeitseinlasskanal (37), der mit dem Flüssigkeitsspeicherhohlraum (13) in Verbindung steht, an dem Basiskörper (325) vorgesehen ist und die flüssigkeitsabsorbierende Fläche (343) in Fluidverbindung mit dem Flüssigkeitseinlasskanal (37) steht.

6. Zerstäuber (100) nach Anspruch 5, wobei der Flüssigkeitsspeicherhohlraum (13) einen Umfang des Luftauslasskanals (11) umgibt und der Flüssigkeitseinlasskanal (37) an einer Fläche des Zerstäubungskerns (34) weg von dem Lufteinlass (33) angeordnet ist und sich in einer Richtung parallel zu der axialen Richtung des Luftauslasskanals (11) erstreckt.

7. Zerstäuber (100) nach einem der Ansprüche 1 bis 6, ferner umfassend ein magnetisches Element (50), wobei die Basis (32) eine Bodenwand (324) gegenüber der oberen Wand (321) umfasst und das magnetische Element (50) an der Bodenwand (324) montiert ist.

8. Elektronische Zerstäubungsvorrichtung (200), umfassend den Zerstäuber (100) nach einem der Ansprüche 1 bis 7.

## Revendications

1. Atomiseur (100), comprenant :
un boîtier (10), dans lequel un canal de sortie d'air (11) est formé dans le boîtier (10) ; et
un assemblage d'atomisation (30) assemblé dans le boîtier (10) et comprenant une base (32) et un noyau d'atomisation (34) inséré dans la base (32), dans lequel la base (32) comprend une paroi de sommet (321) faisant face au canal de sortie d'air (11) et une paroi latérale (323) se recoupant et connectée avec la paroi de sommet (321) ;
dans lequel un canal d'atomisation (35) en communication avec le canal de sortie d'air (11) est défini entre la paroi latérale (323) et le noyau d'atomisation (34), le canal d'atomisation (35) est situé dans une direction axiale du canal de sortie d'air (11), et une entrée d'air (33) en communication avec le canal d'atomisation (35) est prévue ;
dans lequel le noyau d'atomisation (34) comprend une surface d'atomisation (341) parallèle à la direction axiale du canal de sortie d'air (11), la surface d'atomisation (341) est relativement espacée par rapport à la paroi latérale (323), et le canal d'atomisation (35) est défini entre la surface d'atomisation (341) et la paroi latérale (323) ;
dans lequel l'assemblage d'atomisation (30) comprend en outre une électrode (36), le noyau d'atomisation (34) comprend en outre un élément de chauffage, une extrémité de l'électrode (36) est connectée électriquement à l'élément de chauffage, et l'autre extrémité de l'électrode (36) pénètre à travers la paroi latérale (323) et est relativement exposée sur une surface extérieure de la paroi latérale (323) ;
dans lequel la base (32) comprend un corps de base (325) et un support (327), le noyau d'atomisation (34) et le support (327) sont tous les deux insérés dans le corps de base (325), une extrémité du support (327) vient en butée contre le noyau d'atomisation (34), l'autre extrémité du support (327) est la paroi latérale (323),
**caractérisé en ce que**
l'entrée d'air (33) en communication avec le canal d'atomisation (35) est prévue sur la paroi latérale (323), et l'électrode (36) pénètre de manière fixe à travers l'extrémité du support (327) dotée de l'entrée d'air (33).

2. Atomiseur (100) selon la revendication 1, dans lequel l'entrée d'air (33) fait face directement à la surface d'atomisation (341).

3. Atomiseur (100) selon la revendication 1, dans lequel l'électrode (36) pénètre à travers la paroi latérale (323) dans une direction se recoupant avec la direction axiale du canal de sortie d'air (11).

4. Atomiseur (100) selon la revendication 1, dans lequel une cavité d'accueil (40) est définie entre le boîtier (10) et la surface extérieure de la paroi latérale (323), dans lequel la cavité d'accueil (40) est configurée pour loger une portion convexe d'accueil (211) d'une base de cellule (210).

5. Atomiseur (100) selon la revendication 1, dans lequel le noyau d'atomisation (34) comprend une surface d'absorption de liquide (343) en éloignement de la surface d'atomisation (341), une cavité de stockage de liquide (13) est formée à l'intérieur du boîtier (10), un canal d'entrée de liquide (37) en communication avec la cavité de stockage de liquide (13) est prévu sur le corps de base (325), et la surface d'absorption de liquide (343) est en communication fluidique avec le canal d'entrée de liquide (37).

6. Atomiseur (100) selon la revendication 5, dans lequel la cavité de stockage de liquide (13) entoure une périphérie du canal de sortie d'air (11), et le canal d'entrée de liquide (37) est situé sur une surface du noyau d'atomisation (34) en éloignement de l'entrée d'air (33) et s'étend dans une direction parallèle à la direction axiale du canal de sortie d'air (11).

7. Atomiseur (7) selon l'une quelconque des revendications 1 à 6, comprenant en outre un élément magnétique (50), dans lequel la base (32) comprend une paroi de fond (324) opposée à la paroi de sommet (321), et l'élément magnétique (50) est assemblé sur la paroi de fond (324).

8. Dispositif d'atomisation électronique (200), comprenant l'atomiseur (100) selon l'une quelconque des revendications 1 à 7.
